# EUROPÄISCHE PATENTSCHRIFT

(11) **EP 4 040 807 B1**
(45) Veröffentlichungstag und Bekanntmachung des Hinweises auf die Patenterteilung: **26.02.2025**
(21) Anmeldenummer: 22152128.9
(22) Anmeldetag: 19.01.2022
(51) Int. Cl.: H04R 25/00

(54) **HÖRVORRICHTUNGSSYSTEM UND VERFAHREN ZUM BETRIEB EINES HÖRVORRICHTUNGSSYSTEMS**
HEARING AID AND METHOD FOR OPERATING A HEARING AID
SYSTÈME DE DISPOSITIF AUDITIF ET PROCÉDÉ DE FONCTIONNEMENT D'UN SYSTÈME DE DISPOSITIF AUDITIF

(30) Priorität: 03.02.2021 DE 102021200984
(43) Veröffentlichungstag der Anmeldung: 10.08.2022
(73) Patentinhaber: Sivantos Pte. Ltd., Singapore 539775 (SG)
(72) Erfinder: NAUMANN, Frank, 91088 Bubenreuth (DE)
(74) Vertreter: FDST Patentanwälte

(56) Entgegenhaltungen:
- WO-A1-2019/232042
- US-A1- 2019 253 793
- US-A1- 2020 025 732

## Beschreibung

Die Erfindung betrifft ein Hörvorrichtungssystem. Außerdem betrifft die Erfindung ein Verfahren zum Betrieb eines solchen Hörvorrichtungssystems.

Hörvorrichtungen dienen üblicherweise zur Ausgabe eines Tonsignals an das Gehör des Trägers dieser Hörvorrichtung. Die Ausgabe erfolgt dabei mittels eines Ausgabewandlers, meist auf akustischem Weg über Luftschall mittels eines Lautsprechers (auch als "Hörer" oder "Receiver" bezeichnet). Häufig kommen derartige Hörvorrichtungen dabei als sogenannte Hörhilfegeräte (auch kurz: Hörgeräte) zum Einsatz. Dazu umfassen die Hörvorrichtungen normalerweise einen akustischen Eingangswandler (insbesondere ein Mikrophon) und einen Signalprozessor, der dazu eingerichtet ist, das von dem Eingangswandler aus dem Umgebungsschall erzeugte Eingangssignal (auch: Mikrofonsignal) unter Anwendung mindestens eines üblicherweise nutzerspezifisch hinterlegten Signalverarbeitungsalgorithmus derart zu verarbeiten, dass eine Hörminderung des Trägers der Hörvorrichtung zumindest teilweise kompensiert wird. Insbesondere im Fall eines Hörhilfegeräts kann es sich bei dem Ausgabewandler neben einem Lautsprecher auch alternativ um einen sogenannten Knochenleitungshörer oder ein Cochlea-Implantat handeln, die zur mechanischen oder elektrischen Einkopplung des Tonsignals in das Gehör des Trägers eingerichtet sind. Unter dem Begriff Hörvorrichtungen fallen zusätzlich insbesondere auch Geräte wie z.B. sogenannte Tinnitus-Masker, Headsets, Kopfhörer und dergleichen.

Vergleichbar zur fortschreitenden Ausbreitung der Nutzung von Funktionen von sogenannten "wearables", bspw. in Form von Fitnessarmbändern, "smartwatches" und dergleichen, die unter anderem mittels Sensoren Körperfunktionen (bspw. Puls, Bewegung und dergleichen) erfassen, hält auch im Bereich der Hörvorrichtungen die Nutzung von derartigen Funktionen Einzug. Bspw. sollen die Körpertemperatur, der Puls oder dergleichen erfasst werden. Eine solche Zusatznutzung von Hörvorrichtungen bietet sich auch an, da, insbesondere im Fall von Hörhilfegeräten, Hörvorrichtungen auch meist körpernah und häufig auch über einen vergleichsweise langen Zeitraum oder sogar ständig getragen werden.

In US 2019/0253793 A1 wird ein Im-Ohr-Hörgerät offenbart. Das Gerät enthält mindestens einen elektroakustischen Wandler und mindestens einen Sensor oder mindestens eine aktive elektronische Komponente. Der mindestens eine elektroakustische Wandler umfasst eine Kapsel, die einen schallaktiven Teil des Wandlers und ein Luftvolumen des Wandlers umschließt. Das Wandlerluftvolumen ist ein Luftvolumen, das von der Kapsel umschlossen ist und in Fluidverbindung mit dem schallaktiven Teil des Wandlers steht. Mindestens ein Teil des mindestens einen Sensors oder des mindestens einen aktiven elektronischen Bauteils befindet sich innerhalb des Schallwandler-Luftvolumens.

US 2020/0025732 A1 beschreibt die Verwendung von Sensoren an Ohrstöpseln und/oder Kopfhörern zur Überwachung der biometrischen Daten eines Benutzers und/oder der Umgebung.

WO 2019/232042 A1 beschreibt eine biometrische Sensorkopfbaugruppe. Diese umfasst ein Körperelement mit einem inneren Durchgang. Eine optische Quelle ist an einer ersten Stelle an dem Gehäuseelement befestigt, und ein optischer Detektor ist an einer zweiten Stelle an dem Gehäuseelement befestigt. Ein Audiotreiber ist an dem Körperelement befestigt. Ein Ohrgel, das so gestaltet ist, dass es in einen Gehörgang eingeführt werden kann, ist an dem Körperelement befestigt und zumindest ein Teil davon ist für eine oder mehrere Wellenlängen des Lichts transparent. Ein erster Lichtleiter ist an einer Innenfläche des Ohrgels über ein entsprechendes erstes Ende befestigt, und ein zweiter Lichtleiter ist an der Innenfläche des Ohrgels über ein entsprechendes erstes Ende befestigt. Ein zweites Ende des ersten Lichtleiters steht in optischer Verbindung mit der optischen Quelle, ein zweites Ende des zweiten Lichtleiters steht in optischer Verbindung mit dem optischen Detektor.

Der Erfindung liegt die Aufgabe zugrunde, die Erfassung von Körperfunktionen mittels einer Hörvorrichtung zu verbessern.

Diese Aufgabe wird erfindungsgemäß gelöst durch eine Hörvorrichtung mit den Merkmalen des Anspruchs 1. Außerdem wird diese Aufgabe erfindungsgemäß gelöst durch ein Verfahren mit den Merkmalen das Anspruchs 9. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Hörvorrichtungssystem weist einen Photoplethysmographie-Sensor auf, der in einem bestimmungsgemäßen Tragezustand im Gehörgang der das Hörvorrichtungssystem nutzenden Person zu tragen ist. Des Weiteren weist das Hörvorrichtungssystem ein Ohrstück auf, das im bestimmungsgemäßen Tragezustand den Photoplethysmographie-Sensor zumindest teilweise zum Gehörgang hin überdeckt. Insbesondere dient das Ohrstück dazu, den Photoplethysmographie-Sensor zumindest mittelbar in einer bestimmungsgemäßen Trageposition im Gehörgang zu halten. Außerdem weist das Hörvorrichtungssystem einen Controller auf, der dazu eingerichtet ist, anhand von Licht, das mittels des Photoplethysmographie-Sensors erfasst (und vorzugsweise auch ausgestrahlt) wird, eine für eine Transmission des Ohrstücks charakteristische Vergleichsgröße für einen Wellenlängenbereich, der von dem Photoplethysmographie-Sensor genutzt wird, abzuleiten. Des Weiteren ist der Controller dazu eingerichtet, die Vergleichsgröße mit einem vorgegebenen ersten Grenzwert zu vergleichen und, wenn der Grenzwert durch die Vergleichsgröße überschritten wird, eine Warnmeldung auszugeben.

Gemäß dem erfindungsmäßen Verfahren wird zum Betrieb der vorstehend beschriebenen Hörvorrichtung - die also den im bestimmungsgemäßen Tragezustand im Gehörgang zu tragenden Photoplethysmographie-Sensor und das Ohrstück, das im bestimmungsgemäßen Tragezustand den Photoplethysmographie-Sensor zumindest teilweise zum Gehörgang hin überdeckt, aufweist - anhand des mittels des Photoplethysmographie-Sensors erfassten Lichts die Vergleichsgröße für die Transmission des Ohrstücks für einen Wellenlängenbereich des Photoplethysmographie-Sensors abgeleitet, die Vergleichsgröße mit einem vorgegebenen ersten Grenzwert verglichen, und wenn der Grenzwert durch die Vergleichsgröße überschritten wird, eine Warnmeldung ausgegeben.

Das Hörvorrichtungssystem, konkret dessen Controller ist also dazu eingerichtet, das erfindungsgemäß Verfahren, insbesondere selbsttätig, auszuführen. Entsprechend umgekehrt umfasst das Verfahren alle hier und im Folgenden beschriebenen, durch den Controller durchgeführten Maßnahmen. Somit teilen das Hörvorrichtungssystem und das Verfahren die hier und im Folgenden beschriebenen (insbesondere verfahrenstechnischen) Merkmale und die sich daraus ergebenden Vorteile in entsprechender Weise.

Charakteristisch bedeutet hier und im Folgenden, dass die Vergleichsgröße eine quantitative Information über die Größe der Transmission beinhaltet, so dass sich die Transmission zumindest mitteilbar aus der Vergleichsgröße ablesen lässt. So gibt die Vergleichsgröße bspw. die Transmission (insbesondere die Transmission über den gesamten Übertragungsweg des Lichts des Photoplethysmographie-Sensors) selbst oder alternativ auch die zur Transmission komplementäre Absorption an. Bei der Vergleichsgröße kann es sich auch um eine Größe handeln, die zu der anzuzeigenden Transmission direkt oder indirekt proportional ist. Ferner kann die Vergleichsgröße mit der anzuzeigenden Transmission auch in einer nichtlinearen, beispielsweise einer logarithmischen, exponentiellen oder polynomialen (also quadratischen, kubischen, etc.) Beziehung stehen.

Der Begriff "Grenzwert-Überschreitung" (hier die Überschreitung des ersten Grenzwerts) ist hier und im Folgenden vorzugsweise stets richtungsunabhängig in dem Sinne zu verstehen, dass die Differenz der Vergleichsgröße (oder deren zeitliche Änderung) und des Grenzwertes das Vorzeichen wechselt. Je nach Definition der Vergleichsgröße ("echte" Transmission oder Absorption) kann die Überschreitung des jeweiligen Grenzwerts also positiv (im Sinne einer echten Überschreitung, bei der die Vergleichsgröße größer wird als der oder Grenzwert) oder negativ (im Sinne einer Unterschreitung, bei der die Vergleichsgröße kleiner wird als der Grenzwert) sein. So wird die Warnmeldung folglich insbesondere ausgegeben, wenn die Vergleichsgröße die Absorption widerspiegelt und größer wird als der erste Grenzwert. Entsprechend umgekehrt wird die Warnmeldung vorzugsweise ausgegeben, wenn die Vergleichsgröße die Transmission widerspiegelt und unter den ersten Grenzwert absinkt.

Da das Messprinzip des Photoplethysmographie-Sensors darauf beruht, dass Körpergewebe mit Licht aus einem oder mehreren vorgegebenen Wellenlängenbereichen (bspw. unterschiedlichen Bändern des Nahinfrarot-Bereichs und/oder des sichtbaren Wellenlängenbereichs) bestrahlt wird und reflektierte oder transmittierte Strahlung erfasst und zur Bestimmung von Gewebeeigenschaften, insbesondere der aktuellen Durchblutung, herangezogen wird, beeinflusst die Transmission des Ohrstücks offensichtlich die Auswertung und Bestimmung der Gewebeeigenschaften. Vorzugsweise ist deshalb der vorstehend beschriebene erste Grenzwert derart gewählt (vorgegeben), dass für die Auswertung und Bestimmung der Gewebeeigenschaften noch eine hinreichende Lichtmenge empfangen wird. Mit anderen Worten gibt der erste Grenzwert vorzugsweise die Grenze an, ab der eine verlässliche Auswertung und Bestimmung der Gewebeeigenschaften nicht mehr möglich ist. Somit kann also durch die Bestimmung und den Vergleich der Vergleichsgröße mit dem ersten Grenzwert der Nutzer des Hörvorrichtungssystems vorteilhafterweise darüber in Kenntnis gesetzt werden, dass die Funktion des Photoplethysmographie-Sensors eingeschränkt ist.

Der Controller ist weiter dazu eingerichtet, die Vergleichsgröße zusätzlich zu dem ersten Grenzwert auch mit einem zweiten Grenzwert zu vergleichen und in Abhängigkeit von einer Lage der Vergleichsgröße zu dem zweiten Grenzwert auf einen ersten Typ, insbesondere einen ersten Farbtyp, des Ohrstücks zu schließen. Dies ist insbesondere dahingehend zweckmäßig, dass für Hörvorrichtungen oftmals Ohrstücke mit unterschiedlicher Farbgebung (also unterschiedliche Farbtypen) verfügbar sind. Jedoch eignen sich nicht alle Farbgebungen - selbst, wenn das Ohrstück weiterhin transluzent ist - zur verlässlichen (d. h. hinreichend genaue Ergebnisse liefernde) Nutzung des Photoplethysmographie-Sensors, da zumindest ein signifikanter Anteil an Licht absorbiert wird.

**In** einer zweckmäßigen Weiterbildung der vorstehend beschriebenen Ausführung ist der Controller dazu eingerichtet, die Vergleichsgröße mit einem dritten Grenzwert zu vergleichen. Der erste Grenzwert liegt in diesem Fall zwischen dem zweiten und dem dritten Grenzwert. Der Controller ist dabei insbesondere dazu eingerichtet, in Abhängigkeit von einer Lage der Vergleichsgröße zu dem dritten Grenzwert auf einen zweiten Typ, insbesondere einen zweiten Farbtyp, des Ohrstücks zu schließen. Insbesondere ist also der Controller dazu eingerichtet, zwischen einem Farbtyp, der eine hinreichend hohe Transmission aufweist, und einem Farbtyp, der eine zu geringe Transmission aufweist, zu unterscheiden. Dies ist insbesondere dahingehend vorteilhaft, dass für Hörvorrichtungen oftmals unterschiedliche Ohrstücke mit unterschiedlichen Farbgebungen (also unterschiedliche Farbtypen) verfügbar sind, bspw. hell, dunkel, transparent, opak oder dergleichen. Vorzugsweise ist der Controller dazu eingerichtet, bei Detektion eines für die Nutzung des Photoplethysmographie-Sensors ungeeigneten Typs, insbesondere Farbtyps, des Ohrstücks eine Empfehlung für einen geeigneten Typ auszugeben.

Beispielsweise liegt der zweite Grenzwert (für den Fall, dass dieser und somit auch die Vergleichsgröße die Transmission selbst widerspiegeln) oberhalb des ersten Grenzwerts (steht also für einen höheren Wert der Transmission selbst als der durch den ersten Grenzwert gesetzte Wert). In diesem Fall liegt der dritte Grenzwert insbesondere unterhalb des ersten Grenzwerts und steht somit für eine Transmission, die deutlich zu niedrig ist.

Optional sind auch mehrere zweite und/oder dritte Grenzwerte vorgegebenen, anhand derer bspw. mehrere Farbgebungen oder auch mehrere Ohrstücke unterschiedlicher Hersteller unterschieden werden können.

**In** einer zweckmäßigen Ausführung ist der Controller dazu eingerichtet, eine Überschreitung des ersten Grenzwerts durch die Vergleichsgröße als Hinweis auf eine Verschmutzung des Ohrstücks heranzuziehen und mit der Warnmeldung eine Aufforderung zur Reinigung oder Erneuerung des Ohrstücks auszugeben.

In einer vorteilhaften Kombination der Erkennung einer Verschmutzung mit dem vorstehend beschriebenen Vergleich mit dem zweiten oder auch mit dem dritten Grenzwert ist der Controller zweckmäßigerweise dazu eingerichtet, bei Überschreiten nur des ersten Grenzwerts, nicht aber des zweiten oder dritten Grenzwerts, auf die Verschmutzung zu schließen. Eine Detektion der Verschmutzung ist dabei vorteilhafterweise möglich, wenn die Transmission des für die Nutzung des Photoplethysmographie-Sensors ungeeigneten Farbtyps des Ohrstücks derart gering (anders ausgedrückt deren Absorption so hoch) ist, dass der Abstand des ersten Grenzwerts, ab dem die Nutzung des Photoplethysmographie-Sensors signifikant beeinflusst wird. In diesem Fall führt folglich nicht nur ein solcher ungeeigneter Farbtyp sondern auch eine Verschmutzung zur Ausgabe der Warnmeldung.

In einer bevorzugten Ausführung weist das Hörvorrichtungssystem eine Hörvorrichtung (insbesondere ein Hörhilfegerät) auf, mit der der Photoplethysmographie-Sensor gekoppelt ist. Insbesondere ist der Photoplethysmographie-Sensor Teil der Hörvorrichtung. Der Controller ist in diesem Fall zweckmäßigerweise dazu eingerichtet, die Vergleichsgröße nach einem Wechsel des Ohrstücks und/oder nach einer Aktivierung der Hörvorrichtung zu ermitteln, sowie vorzugsweise auch mit dem oder dem jeweiligen Grenzwert zu vergleichen. Optional ist der Controller dazu eingerichtet, auch während des laufenden Betriebs der Hörvorrichtung wiederkehrend die Vergleichsgröße zu ermitteln und insbesondere auch mit dem oder dem jeweiligen Grenzwert zu vergleichen.

In einer zweckmäßigen Ausführung ist der erste Grenzwert, sowie gegebenenfalls auch der zweite und/oder der dritte Grenzwert, derart gewählt, dass ein Einfluss von Körpergewebe auf das ausgestrahlte Licht des Photoplethysmographie-Sensors berücksichtigt ist. Anders ausgedrückt ist der entsprechende Grenzwert bereits so gewählt, dass die Vergleichsgröße nach Bestrahlung des Körpergewebes ermittelt wird. Erkanntermaßen erfolgt durch das Körpergewebe eine (zum Ohrstück zusätzliche) "Dämpfung" des ausgestrahlten Lichts. Der erste Grenzwert gibt mithin vorzugsweise an, wie viel Lichtmenge für die ordnungsgemäße, insbesondere verlässliche, Nutzung des Photoplethysmographie-Sensors nach Bestrahlung des Körpers des Nutzers erfasst werden muss. Entsprechend geben der zweite bzw. dritte Grenzwert - soweit vorhanden - an, wie viel Lichtmenge nach der Bestrahlung des Körpers erfasst werden muss, um dem Ohrstück den jeweiligen Farbtyp zuzuweisen.

Vorzugsweise bildet der erste (insbesondere auch der zweite und dritte) Grenzwert das Verhältnis von empfangender Lichtmenge (Intensität) zu abgestrahlter Lichtmenge ab. Insbesondere beträgt der erste Grenzwert mindestens 65 Prozent bei einer Wellenlänge im Bereich um 860 (+/- 20) Nanometer.

In einer weiteren zweckmäßigen Ausführung umfasst die Hörvorrichtung einen hinter dem Ohr zu tragenden Hauptkörper, der einen Signalprozessor und wenigstens ein mit diesem gekoppeltes Mikrofon umfasst. Des Weiteren umfasst die Hörvorrichtung in diesem Fall einen im Gehörgang zu tragenden, signalübertragungstechnisch mit dem Signalprozessor gekoppelten Lautsprecher. Insbesondere handelt es sich bei der Hörvorrichtung um ein Hinter-dem-Ohr-Hörhilfegerät (auch als "BTE" für "behind the ear" bezeichnet) mit externem Lautsprecher (unter anderem auch als "RIC" für "receiver in canal" oder "RIC-BTE" bezeichent). **In** dieser Ausführung trägt vorzugsweise der Lautsprecher den Photoplethysmographie-Sensor, insbesondere ist letzterer in den Lautsprecher integriert.

Insbesondere im vorstehend genannten Fall des "RIC"-Hörhilfegeräts ist das (insbesondere zur Nutzung mit dem den Photoplethysmographie-Sensor geeignete oder vorgesehene) Ohrstück bevorzugt als flexibler Überzug für den Lautsprecher und aus transparentem, insbesondere nicht eingefärbtem Material gebildet. Beispielsweise ist das Ohrstück - in diesem Fall auch als "ear dome" bezeichnet - aus einem Silikon gebildet. Ein nicht zur Nutzung mit dem den Photoplethysmographie-Sensor geeignetes Ohrstück ist dagegen bspw. aus einem (insbesondere dunkel) gefärbten Material, insbesondere Silikon, gebildet.

Der vorstehend beschriebene Controller des Hörvorrichtungssystems ist optional als nicht-programmierbare elektronische Schaltung ausgebildet. Alternativ ist der Controller durch einen Mikrocontroller gebildet, in dem die Funktionalität zur Durchführung des vorstehend beschriebenen, erfindungsgemäßen Verfahrens in Form eines Softwaremoduls implementiert ist.

Der Controller ist in einer optionalen Ausführung in den Signalprozessor der Hörvorrichtung integriert. In einer alternativen oder optional auch zusätzlichen (bspw. um wahlweise einen für die Hörvorrichtung energiesparenderen Modus aufnehmen zu können) Ausführung ist der Controller extern von der Hörvorrichtung ausgebildet und bspw. auf einem Smartphone als App (d. h. das vorstehend genannte Softwaremodul) implementiert. Das Smartphone stellt somit insbesondere bei installierter App zumindest während der Nutzung der App einen Teil des Hörvorrichtungssystems dar. In diesem Fall werden die Daten (insbesondere die Sensorsignale) des Photoplethysmographie-Sensors vorzugsweise drahtlos mittels einer Kommunikationsschnittstelle der Hörvorrichtung auf das Smartphone übertragen.

Nachfolgend werden Ausführungsbeispiele der Erfindung anhand einer Zeichnung näher erläutert. Darin zeigen:
- Fig. 1: in einer schematischen Darstellung ein Hörvorrichtungssytem mit einer Hörvorrichtung, die einen externen Lautsprecher und einen Photoplethysmographie-Sensor aufweist,
- Fig. 2: in einer schematischen Teilschnittansicht den Lautsprecher mit dem Photoplethysmographie-Sensor im bestimmungsgemäßen Tragezustand im Gehörgang eines Nutzers, und
- Fig. 3, 4: jeweils in schematischen Diagrammen eine im bestimmungsgemäßen Tragezustand mittels des Photoplethysmographie-Sensors für eine Transmission eines Ohrstücks ermittelte Vergleichsgröße.

Einander entsprechende Teile und Größen sind in allen Figuren stets mit gleichen Bezugszeichen versehen.

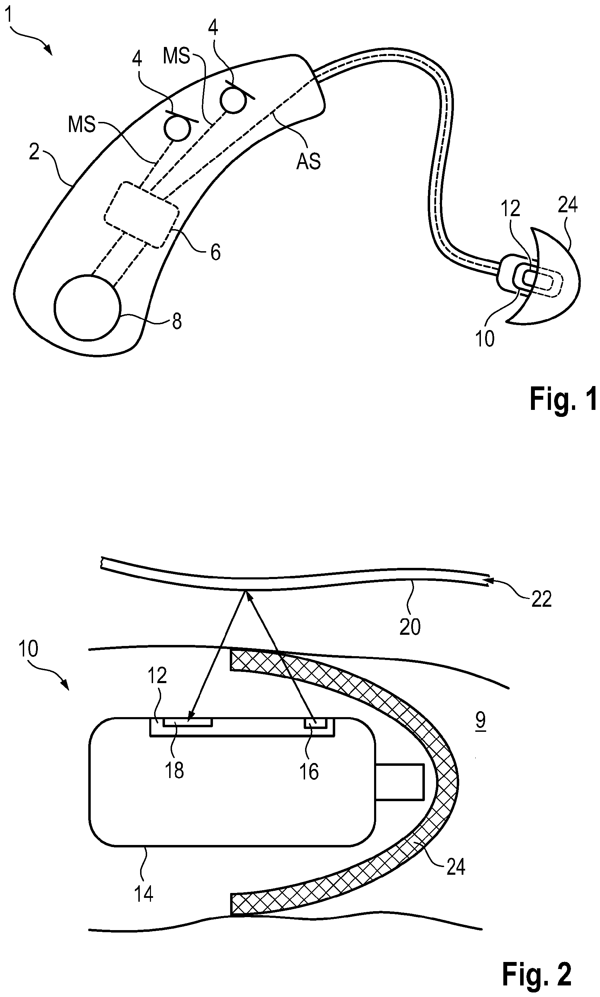

In Fig. 1 ist eine Hörvorrichtung in Form eines Hörhilfegeräts, konkret eines hinter dem Ohr eines Nutzers zu tragenden Hörhilfegeräts (kurz auch als Hörgerät, hier als "BTE 1" bezeichnet), dargestellt. Das BTE 1 umfasst ein Gehäuse 2, in dem elektronische Komponenten des BTE 1 angeordnet sind. Bei diesen elektronischen Komponenten handelt es sich beispielsweise um zwei Mikrofone 4, einen Signalprozessor 6 und ein Batteriemodul 8. Die Mikrofone 4 dienen im bestimmungsgemäßen Betrieb des BTE 1 zum Empfang von Umgebungsschall und Wandlung dessen in elektrische Eingangssignale (auch: "Mikrofonsignale MS"), die von dem Signalprozessor 6 (auch als "Controller" bezeichnet) verarbeitet (insbesondere gefiltert, frequenzabhängig verstärkt und/oder gedämpft etc.) werden. Die verarbeiteten Eingangssignale werden anschließend als Ausgangssignale AS an einen extern zum Gehäuse 2 angeordneten und bestimmungsgemäß im Gehörgang 9 zu tragenden Lautsprecher 10 ausgegeben und von diesem in Schallsignale gewandelt und an das Gehör des Nutzers weitergegeben.

Das BTE 1 weist außerdem einen Photoplethysmographie-Sensor, kurz "PPG-Sensor 12" auf, der im vorliegenden Ausführungsbeispiel in den Lautsprecher 10 integriert ist, konkret in dessen Gehäuse 14 (s. Fig. 2) eingesetzt ist. Der PPG-Sensor 12 dient dazu, bspw. den Puls, optional auch die Sauerstoffsättigung des Nutzers des BTE 1 zu ermitteln. Dazu umfasst der PPG-Sensor 12 eine Lichtquelle, im vorliegenden Fall eine LED-Einheit 16, die dazu eingerichtet ist, mehrere Frequenzbänder, üblicherweise aber zumindest Licht im Nah-Infrarot-Bereich, auszugeben. Außerdem umfasst der PPG-Sensor 12 einen Lichtsensor 18, mittels dessen eingestrahltes Licht erfasst wird. Im bestimmungsgmäßen Betrieb erfasst der Lichtsensor 18 Strahlung, die von der LED-Einheit 16 ausgegeben und von Körpergewebe, bspw. einer Gefäßwand 20 eines Blutgefäßes 22 des Nutzers reflektiert wird. Anhand des dabei erfassten Intensitätsverlaufs kann dann bspw. der Puls des Nutzers bestimmt werden.

Um den Lautsprecher 10 im Gehörgang 9 zu halten, ist das BTE 1 außerdem mit einem Ohrstück bestückt, hier konkret durch einen als "dome 24" (oder auch: "sleeve") bezeichneten, pilzartigen und flexiblen Überzug gebildet. Dieser dome 24 überdeckt, wie in Fig. 1 und 2 zu erkennen ist, den PPG-Sensor 12 zumindest teilweise. Aufgrund der dem Material des domes 24 inhärenten Absorption (d. h. einer Transmission von weniger als 100 Prozent) wird also bereits das Licht auf seinem Weg zwischen der LED-Einheit 16 und dem Lichtsensor 18 gedämpft. Abhängig von der Farbgebung des domes 24 ist diese Dämpfung mehr oder weniger stark. Für das BTE 1 sind üblicherweise unterschiedlich gefärbte domes 24, meist eine ungefärbte und eine dunkle Variante, verfügbar.

Das BTE 1 bildet gemeinsam mit dem aktuell applizierten dome 24 auch ein "Hörvorrichtungssystem".

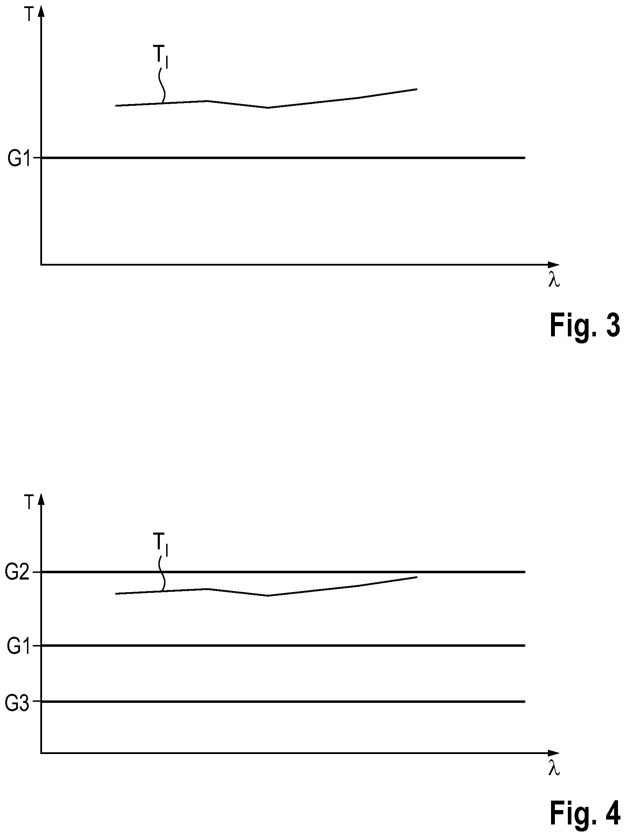

Allerdings ist für eine bestimmungsgemäße Nutzung, konkret für verlässliche Ergebnisse des PPG-Sensors 12, ein vorgegebener Intensitätswert, der mittels des Lichtsensors 18 mindestens empfangen wird, erforderlich. Der Signalprozessor 6 ist deshalb dazu eingerichtet, anhand des erfassten Lichts (optional für jedes ausgestrahlte Wellenlängenband oder nur beispielhaft für ein Band) eine Vergleichsgröße zu ermitteln, die charakteristisch für die Transmission des domes 24 ist. Diese Vergleichsgröße - hier als "Transmissionsistwert TI" bezeichnet - wird dabei als Verhältnis zwischen der erfassten Intensität des Lichts zur von der LED-Einheit 16 ausgestrahlten Intensität ermittelt. Dieser Transmissionsistwert TI beinhaltet somit erkanntermaßen sowohl die Dämpfung durch den dome 24 als auch durch das Körpergewebe des Nutzers. Fig. 3 zeigt ein Diagramm, in dem die Transmission T gegen die Wellenlänge λ angetragen ist. Hierbei ist der Verlauf des Transmissionsistwerts TI über die erfassten Wellenlängenbänder beispielhaft dargestellt.

Der Signalprozessor 6 vergleicht anschließend den Transmissionsistwert TI mit einem vorgegebenen ersten Grenzwert G1 (s. Fig. 3). Dieser erste Grenzwert G1 ist derart gewählt, dass unterhalb eine verlässliche Funktion des PPG-Sensors 12 nicht gegeben ist, also die Dämpfung (Absorption) durch den dome 24 und das Körpergewebe zu hoch ist. Liegt der Transmissionsistwert TI, wie in Fig. 3 dargestellt, oberhalb der ersten Grenzwerts G1 führt der Signalprozessor 6 den bestimmungsgemäßen Betrieb fort. Unterschreitet der Transmissionsistwert TI den Grenzwert G1, gibt der Signalprozessor 6 eine Warnmeldung aus.

Um die Warnmeldung weiter spezifizieren zu können, sind in einem weitere Ausführungsbeispiel in dem Signalprozessor 6 ein zweiter Grenzwert G2 und ein dritter Grenzwert G3 abgespeichert. Der zweite Grenzwert G2 liegt hierbei oberhalb und der dritte Grenzwert G3 unterhalb des ersten Grenzwerts G1.

Dadurch kann der Signalprozessor 6 auch unterschiedliche Typen, konkret unterschiedliche Farben, hier konkret eine helle oder farblose und eine dunkle Farbe, des domes 24 unterscheiden. Unterschreitet der Transmissionsistwert **TI** den ersten und den dritten Grenzwert G1 und G3, schließt der Signalprozessor 6 darauf, dass die dunkle Farbvariante des domes 24 montiert ist. Der Signalprozessor 6 gibt mit der Warnmeldung die Anweisung aus, die ungefärbte Variante des domes 24 zu montieren.

Überschreitet der Transmissionsistwert **TI** auch den zweiten Grenzwert G2, schließt der Signalprozessor 6 darauf, dass die ungefärbte Variante des domes 24 montiert ist.

Für den Fall, dass der Transmissionsistwert **TI** den ersten Grenzwert G1, aber nicht den dritten Grenzwert G3 unterschreitet, schließt der Signalprozessor 6 in einem weiteren Ausführungsbeispiel darauf, dass der dome 24 verschmutzt ist, bspw. da sich Cerumen am dome 24 angelagert hat. **In** diesem Fall gibt der Signalprozessor 6 mit der Warnmeldung die Empfehlung aus, den dome 24 zu reinigen oder zu ersetzen.

**In** einem nicht näher dargestellten Ausführungsbeispiel umfasst das vorstehend genannte Hörvorrichtungssystem außerdem ein Smartphone, auf dem eine Softwareapplikation implementiert ist, die zur Auswertung der Daten des PPG-Sensors 12 eingerichtet und vorgesehen ist. Diese Applikation ist in diesem Ausführungsbeispiel auch dazu eingerichtet und vorgesehen, die vorstehend durch den Signalprozessor 6 durchgeführte "Überwachung" der Transmission des domes 24 durchzuführen. **In** diesem Fall stellt also das Smartphone, konkret dessen Mikroprozessor in Zusammenspiel mit der Applikation einen Controller dar, der den Transmissionsistwert TI bestimmt und diesen mit dem ersten Grenzwert G1 und gegebenenfalls auch mit den zweiten und dritten Grenzwerten G2 und G3 vergleicht, sowie gegebenenfalls die Warnmeldung ausgibt. Das BTE 1 weist in diesem Fall eine Drahtlos-Schnittstelle auf, mittels derer die Daten des PPG-Sensors 12 an das Smartphone übermittelt werden.

### Bezugszeichenliste

- 1: BTE
- 2: Gehäuse
- 4: Mikrofon
- 6: Signalprozessor
- 8: Batteriemodul
- 9: Gehörgang
- 10: Lautsprecher
- 12: PPG-Sensor
- 14: Gehäuse
- 16: LED-Einheit
- 18: Lichtsensor
- 20: Gefäßwand
- 22: Blutgefäß
- 24: dome

- MS: Mikrofonsignal
- AS: Ausgangssignal
- T: Transmission
- TI: Transmissionsistwert
- G1: erster Grenzwert
- G2: zweiter Grenzwert
- G3: dritter Grenzwert
- λ: Wellenlänge

## Patentansprüche

1. Hörvorrichtungssystem, aufweisend
- einen in einem bestimmungsgemäßen Tragezustand im Gehörgang zu tragenden Photoplethysmographie-Sensor (12),
- ein Ohrstück (24), das im bestimmungsgemäßen Tragezustand den Photoplethysmographie-Sensor (12) zumindest teilweise zum Gehörgang (9) hin überdeckt, und
- einen Controller (6),
**dadurch gekennzeichnet,**
**dass** der Controller (6) dazu eingerichtet ist, anhand von mittels des Photoplethysmographie-Sensors (12) erfasstem Licht eine für eine Transmission (T) des Ohrstücks (24) charakteristische Vergleichsgröße (TI) des Ohrstücks (24) für einen Wellenlängenbereich des Photoplethysmographie-Sensors (12) abzuleiten, die Vergleichsgröße (TI) mit einem vorgegebenen ersten Grenzwert (G1) zu vergleichen und, wenn der erste Grenzwert (G1) durch die Vergleichsgröße (TI) überschritten wird, eine Warnmeldung auszugeben, und dass der Controller (6) dazu eingerichtet ist, die Vergleichsgröße (TI) mit einem zweiten Grenzwert (G2) zu vergleichen und in Abhängigkeit von einer Lage der Vergleichsgröße (TI) zu dem zweiten Grenzwert (G2) auf einen ersten Typ, insbesondere einen ersten Farbtyp, des Ohrstücks (24) zu schließen.

2. Hörvorrichtungssystem nach Anspruch 1,
wobei der Controller (6) dazu eingerichtet ist, die Vergleichsgröße (TI) mit einem dritten Grenzwert (G3) zu vergleichen, wobei der erste Grenzwert (G1) zwischen dem zweiten und dem dritten Grenzwert (G2,G3) liegt, und in Abhängigkeit von einer Lage der Vergleichsgröße (TI) zu dem dritten Grenzwert (G3) auf einen zweiten Typ, insbesondere einen zweiten Farbtyp, des Ohrstücks (24) zu schließen.

3. Hörvorrichtungssystem nach einem der Ansprüche 1 bis 2,
wobei der Controller (6) dazu eingerichtet ist, eine Überschreitung des ersten Grenzwerts (G1) durch die Vergleichsgröße (TI) als Hinweis auf eine Verschmutzung des Ohrstücks (24) heranzuziehen und mit der Warnmeldung eine Aufforderung zur Reinigung oder Erneuerung des Ohrstücks (24) auszugeben.

4. Hörvorrichtungssystem nach Anspruch 3 und nach Anspruch 1 oder 2, wobei der Controller (6) dazu eingerichtet ist, bei Überschreiten nur des ersten Grenzwerts (G1), nicht aber des zweiten oder dritten Grenzwerts (G2,G3), auf die Verschmutzung zu schließen.

5. Hörvorrichtungssystem nach einem der Ansprüche 1 bis 4,
aufweisend eine Hörvorrichtung (1), mit der der Photoplethysmographie-Sensor (12) gekoppelt ist, wobei der Controller (6) dazu eingerichtet ist, die Vergleichsgröße (TI) nach einem Wechsel des Ohrstücks (24) und/oder nach einer Aktivierung der Hörvorrichtung (1) zu ermitteln.

6. Hörvorrichtungssystem nach einem der Ansprüche 1 bis 5,
wobei der erste Grenzwert (G1), sowie gegebenenfalls auch der zweite und/oder der dritte Grenzwert (G2,G3), derart gewählt ist, dass ein Einfluss von Körpergewebe auf das ausgestrahlte Licht des Photoplethysmographie-Sensors (12) berücksichtigt ist.

7. Hörvorrichtungssystem nach einem der Ansprüche 1 bis 6,
wobei die Hörvorrichtung (1) einen hinter dem Ohr zu tragenden Hauptkörper, der einen Signalprozessor (6) und wenigstens ein mit diesem gekoppeltes Mikrofon (4) umfasst, und einen im Gehörgang (9) zu tragenden, signal-übertragungstechnisch mit dem Signalprozessor (6) gekoppelten Lautsprecher (10) aufweist, wobei der Lautsprecher (10) den Photoplethysmographie-Sensor (12) trägt.

8. Hörvorrichtungssystem nach Anspruch 7,
wobei das Ohrstück (24) als flexibler Überzug für den Lautsprecher (10) ausgebildet ist und aus transparentem, insbesondere nicht eingefärbtem Material gebildet ist.

9. Verfahren zum Betrieb eines Hörvorrichtungssystems, welches einen in einem bestimmungsgemäßen Tragezustand im Gehörgang (9) zu tragenden Photoplethysmographie-Sensor (12) und ein Ohrstück (24), das im bestimmungsgemäßen Tragezustand den Photoplethysmographie-Sensor (12) zum Gehörgang (9) hin überdeckt, aufweist,
**dadurch gekennzeichnet, dass** verfahrensgemäß
- anhand von mittels des Photoplethysmographie-Sensors (12) erfasstem Licht eine für eine Transmission (T) des Ohrstücks (24) charakteristische Vergleichsgröße (TI) für einen Wellenlängenbereich des Photoplethysmographie-Sensors (12) abgeleitet wird,
- die Vergleichsgröße (TI) mit einem vorgegebenen ersten Grenzwert (G1) verglichen wird,
- wenn der erste Grenzwert (G1) durch die Vergleichsgröße (TI) überschritten wird, eine Warnmeldung ausgegeben wird, und
- die Vergleichsgröße (TI) mit einem zweiten Grenzwert (G2) verglichen wird und in Abhängigkeit von einer Lage der Vergleichsgröße (TI) zu dem zweiten Grenzwert (G2) auf einen ersten Typ, insbesondere einen ersten Farbtyp, des Ohrstücks (24) geschlossen wird.

## Claims

1. Hearing device system, having
- a photoplethysmography sensor (12) to be worn in the auditory canal in an intended worn state,
- an earpiece (24) which at least partly covers the photoplethysmography sensor (12) toward the auditory canal (9) in the intended worn state, and
- a controller (6),
**characterized**
**in that** the controller (6) is configured to use light captured by the photoplethysmography sensor (12) to derive a comparison quantity (TI) of the earpiece (24) characteristic for a transmission (T) of the earpiece (24), for a wavelength range of the photoplethysmography sensor (12), to compare the comparison quantity (TI) with a specified first limit (G1) and to output an alert if the first limit (G1) is traversed by the comparison quantity (TI),
and **in that** the controller (6) is configured to compare the comparison quantity (TI) with a second limit (G2) and to deduce a first type, in particular a first colour type, of the earpiece (24) on the basis of a relative position of the comparison quantity (TI) in relation to the second limit (G2).

2. Hearing device system according to Claim 1, wherein the controller (6) is configured to compare the comparison quantity (TI) with a third limit (G3), the first limit (G1) being between the second and the third limit (G2, G3), and to deduce a second type, in particular a second colour type, of the earpiece (24) on the basis of a relative position of the comparison quantity (TI) in relation to the third limit (G3).

3. Hearing device system according to either one of Claims 1 and 2,
wherein the controller (6) is configured to draw upon the first limit (G1) being traversed by the comparison quantity (TI) as an indication for dirtying of the earpiece (24) and to output a request to clean or renew the earpiece (24) with the alert.

4. Hearing device system according to Claim 3 and according to Claim 1 or 2,
wherein the controller (6) is configured to deduce dirtying if only the first limit (G1) is traversed and not the second or third limit (G2, G3).

5. Hearing device system according to any one of Claims 1 to 4,
having a hearing device (1) to which the photoplethysmography sensor (12) is coupled, wherein the controller (6) is configured to determine the comparison quantity (TI) following a replacement of the earpiece (24) and/or following the activation of the hearing device (1).

6. Hearing device system according to any one of Claims 1 to 5,
wherein the first limit (G1), and optionally also the second and/or the third limit (G2, G3), is chosen such that an influence of body tissue on the light emitted by the photoplethysmography sensor (12) is taken into account.

7. Hearing device system according to any one of Claims 1 to 6,
wherein the hearing device (1) has a main body that is to be worn behind the ear, said main body comprising a signal processor (6) and at least one microphone (4) coupled therewith, and a loudspeaker (10) which is to be worn in the auditory canal (9) and coupled to the signal processor (6) for signal transmission purposes, the loudspeaker (10) carrying the photoplethysmography sensor (12).

8. Hearing device system according to Claim 7,
wherein the earpiece (24) is designed as a flexible cover for the loudspeaker (10) and is formed from transparent, more particularly non-coloured material.

9. Method for operating a hearing device system which has a photoplethysmography sensor (12) to be worn in the auditory canal (9) in an intended worn state and an earpiece (24) which covers the photoplethysmography sensor (12) toward the auditory canal (9) in the intended worn state,
**characterized in that** according to the method
- light captured by the photoplethysmography sensor (12) is used to derive a comparison quantity (TI) characteristic for a transmission (T) of the earpiece (24), for a wavelength range of the photoplethysmography sensor (12),
- the comparison quantity (TI) is compared with a specified first limit (G1),
- an alert is output if the first limit (G1) is traversed by the comparison quantity (TI), and
- the comparison quantity (TI) is compared with a second limit (G2) and a first type, in particular a first colour type, of the earpiece (24) is deduced on the basis of a relative position of the comparison quantity (TI) in relation to the second limit (G2).

## Revendications

1. Système à appareil auditif, comprenant
- un capteur photopléthysmographique (12) destiné, lorsqu'il est porté comme prévu, à être porté dans le conduit auditif,
- un écouteur (24) qui, lorsqu'il est porté comme prévu, recouvre le capteur photopléthysmographique (12) au moins partiellement en direction du conduit auditif (9), et
- un contrôleur (6),
**caractérisé en ce que**
le contrôleur (6) est conçu pour déduire, sur la base de la lumière détectée au moyen du capteur photopléthysmographique (12), une grandeur de comparaison (TI) de l'écouteur (24) qui est caractéristique d'une transmission (T) de l'écouteur (24) pour un domaine de longueurs d'onde du capteur photopléthysmographique (12), comparer la grandeur de comparaison (TI) à une première valeur limite spécifiée (G1) et, si la première valeur limite (G1) est dépassée par la grandeur de comparaison (TI), délivrer un message d'avertissement, et
le contrôleur (6) est conçu pour comparer la grandeur de comparaison (TI) à une deuxième valeur limite (G2) et, en fonction d'une position de la grandeur de comparaison (TI) par rapport à la deuxième valeur limite (G2), à un premier type, en particulier un premier type de couleur, de l'écouteur (24).

2. Système à appareil auditif selon la revendication 1,
le contrôleur (6) étant conçu pour comparer la grandeur de comparaison (TI) à une troisième valeur limite (G3), la première valeur limite (G1) étant située entre la deuxième et la troisième valeur limite (G2, G3), et en fonction d'une position de la grandeur de comparaison (TI) par rapport à la troisième valeur limite (G3), conclure à un deuxième type, notamment un deuxième type de couleur, de l'écouteur (24).

3. Système à appareil auditif selon l'une des revendications 1 à 2,
le contrôleur (6) étant conçu pour utiliser un dépassement de la première valeur limite (G1) par la grandeur de comparaison (TI) comme indication que l'écouteur (24) est encrassé et déliver, à l'aide du message d'avertissement, une demande de nettoyage ou de remplacement de l'écouteur (24).

4. Système à appareil auditif selon la revendication 3 et selon la revendication 1 ou 2,
le contrôleur (6) étant conçu pour conclure à l'encrassement lorsque seule la première valeur limite (G1), mais pas la deuxième ou la troisième valeur limite (G2, G3), est dépassée.

5. Système à appareil auditif selon l'une des revendications 1 à 4, comprenant un appareil auditif (1) auquel le capteur photopléthysmographique (12) est accouplé, le contrôleur (6) étant conçu pour déterminer la grandeur de comparaison (TI) après avoir changé l'écouteur (24) et/ou après avoir activé l'appareil auditif (1).

6. Système à appareil auditif selon l'une des revendications 1 à 5,
la première valeur limite (G1), et éventuellement également la deuxième et/ou la troisième valeur limite (G2, G3), étant sélectionnées de manière à ce qu'une influence des tissus corporels sur la lumière émise par le capteur photopléthysmographique (12) soit prise en compte.

7. Système à appareil auditif selon l'une des revendications 1 à 6,
l'appareil auditif (1) comportant un corps principal qui est destiné à être porté derrière l'oreille et qui comprend un processeur de signal (6) et au moins un microphone (4) qui est accouplé à celui-ci, et un haut-parleur (10) qui est destiné à être porté dans le conduit auditif (9) et qui est accouplé au processeur de signal (6) en termes de transmission du signal, le haut-parleur (10) portant le capteur photopléthysmographique (12).

8. Système à appareil auditif selon la revendication 7,
l'écouteur (24) étant conçu comme un revêtement flexible destiné au haut-parleur (10) et étant formé d'un matériau transparent, en particulier incolore.

9. Procédé de fonctionnement d'un système à appareil auditif qui comporte un capteur photopléthysmographique (12) destiné, lorsqu'il est porté comme prévu, à être porté dans le conduit auditif (9), et un écouteur (24) qui, lorsqu'il est porté comme prévu, recouvre le capteur photopléthysmographique (12) au moins partiellement en direction du conduit auditif (9), **caractérisé en ce que** selon le procédé
- sur la base de la lumière détectée au moyen du capteur photopléthysmographique (12), une grandeur de comparaison (TI) qui est caractéristique d'une transmission (T) de l'écouteur (24) est déduite pour un domaine de longueurs d'onde du capteur photopléthysmographique (12),
- la grandeur de comparaison (TI) est comparée à une première valeur limite spécifiée (G1),
- si la première valeur limite (G1) est dépassée par la grandeur de comparaison (TI), un message d'avertissement est délivré, et
- la grandeur de comparaison (TI) est comparée à une deuxième valeur limite (G2) et, en fonction d'une position de la grandeur de comparaison (TI) par rapport à la deuxième valeur limite (G2), une conclusion est tirée quant à un premier type, en particulier un premier un type couleur, de l'écouteur (24).
